(19) Europäisches Patentamt
European Patent Office
Office européen des brevets

(11) EP 0 735 853 B1

(12) **EUROPÄISCHE PATENTSCHRIFT**

(45) Veröffentlichungstag und Bekanntmachung des Hinweises auf die Patenterteilung:
**20.01.1999 Patentblatt 1999/03**

(21) Anmeldenummer: **94928281.8**

(22) Anmeldetag: **01.10.1994**

(51) Int. Cl.$^6$: **A61K 7/00**

(86) Internationale Anmeldenummer:
**PCT/DE94/01157**

(87) Internationale Veröffentlichungsnummer:
**WO 95/17155 (29.06.1995 Gazette 1995/27)**

(54) **W/O/W-EMULSIONEN**
W/O/W EMULSIONS
EMULSIONS EAU DANS L'HUILE DANS L'EAU

(84) Benannte Vertragsstaaten:
**AT BE CH DE ES FR GB IT LI NL**

(30) Priorität: **22.12.1993 DE 4343833**

(43) Veröffentlichungstag der Anmeldung:
**09.10.1996 Patentblatt 1996/41**

(73) Patentinhaber:
**Beiersdorf Aktiengesellschaft**
**20245 Hamburg (DE)**

(72) Erfinder:
- **GOHLA, Sven**
  **D-21077 Hamburg (DE)**
- **MÜLLER, Anja**
  **D-20535 Hamburg (DE)**
- **NIELSEN, Jens**
  **D-24558 Henstedt (DE)**

(56) Entgegenhaltungen:
**DE-A- 4 136 699**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 735 853 B1

Printed by Xerox (UK) Business Services
2.16.7/3.6

## Beschreibung

Die vorliegende Erfindung betrifft stabile multiple Emulsionen vom W/O/W-Typ, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zubereitungen enthalten in der Regel Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist bekannt, daß sich multiple Emulsionen - unter anderem - durch eine besonders feine Emulsionstextur auszeichnen können. Diese Eigenschaft eignet sie hervorragend als Basis sowohl für kosmetische wie für medizinische topische Zubereitungen. Wo allerdings Kosmetika nur äußerlich angewandt werden, sind bei medizinischer Verwendung von Emulsionen alle üblichen Applikationsarten, z.B. orale Verabreichungsformen, denkbar.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

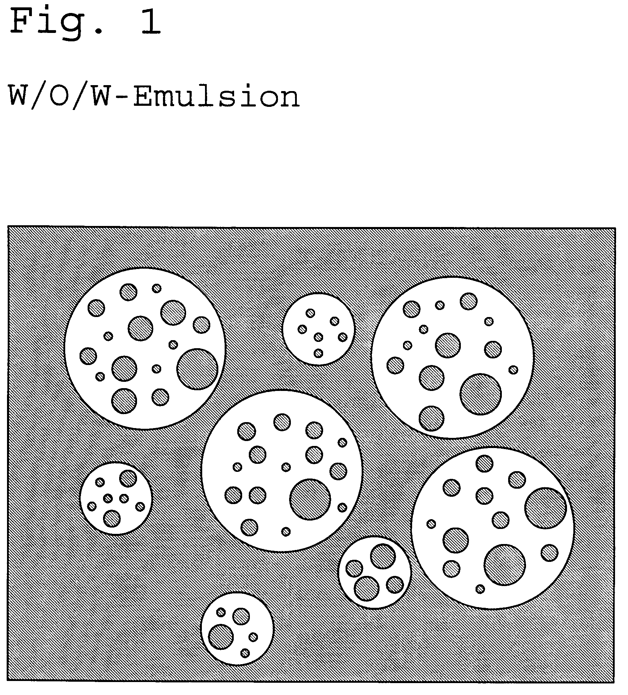

In Figur 1 wird eine W/O/W-Emulsion schematisch dargestellt, wobei die schraffierten Flächen die äußere und innere Wasserphase und die weißen Flächen die Ölphase bedeuten.

Multiple Emulsionen, bei welchen die jeweiligen inneren und äußeren Wasserphasen oder inneren und äußeren Ölphasen unterschiedlich geartet sind (also z.B. W/O/W'- und O/W/O'-Emulsionen), sind der Präparation durch Zweitopfverfahren zugängig. Solche Emulsionen, in welchen die inneren und äußeren Wasser- bzw. Ölphasen nicht unterschiedlich geartet sind, sind sowohl durch Ein- als auch durch Zweitopfverfahren erhältlich.

Die multiplen Emulsionen zweiten Grades werden gelegentlich als "bimultiple Systeme", solche dritten Grades als "trimultiple Systeme" usw., bezeichnet (W.Seifriz, Studies in Emulsions, J.Phys.Chem., 29 (1925) 738 - 749).

Verfahren zur Herstellung multipler Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (z.B. eine Wasserphase) mit einem entsprechenden Emulgator (z.B. ein O/W-Emulgator) in eine multiple Emulsion (z.B. eine W/O/W-Emulsion) überführt wird.

Eine zweites Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Die Definition für den HLB-Wert ist für Polyolfettsäureester gegeben durch die Formel I

$$HLB = 20 * ( 1 - S/A)$$

Für eine Gruppe von Emulgatoren, deren hydrophiler Anteil nur aus Ethylenoxideinheiten besteht, gilt die Formel II

$$HLB = E/5$$

wobei

S = Verseifungszahl des Esters,
A = Säurezahl der zurückgewonnen Säure
E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die US-Patentschrift 4,931,210 beschreibt ein Verfahren zur Herstellung von W/O/W-Emulsion, wobei als Emulgatoren Polyglycerinpolyricinoleate verwendet werden.

Obwohl also multiple Emulsionen an sich bekannt sind und es durchaus einfache Verfahren zu ihrer Herstellung gibt, hat es dennoch bis heute an solchen Systemen gemangelt, welche mikroskopisch über längere Lagerzeiten (beispielsweise über mehrere Jahre) bzw. in einem breiten Temperaturbereich (beispielsweise von -10° C bis +50° C) bzw. gegenüber extremen Temperaturschwankungen stabil (schaukelstabil, beipielsweise von -15 bis +50° C) sind. Dies soll bedeuten, daß sich die multiplen Emulsionen des Standes der Technik mit der Zeit in einfache W/O- oder O/W-Emulsionen umwandeln, also eine im Sinne der Multiplizität geringe Lagerungsstabilität aufweisen. Dies ist insbesondere nachteilig, als diese Umwandlungsprodukte eine äußerst inhomogene Tröpfchengrößenverteilung zur Folge haben.

Bestenfalls sind solche Umwandlungsprodukte unschön oder aus kosmetischer Sicht unelegant. Oft ist jedoch mit der inhomogenen Größenverteilung der Tröpfchen auch mangelnde makroskopische Stabilität verbunden, also die Stabilität gegen die Zersetzung in getrennte Phasen.

Auch in dieser Hinsicht waren die herkömmlichen multiplen Emulsionen stets entweder unzureichend stabil, oder aber die Übertragung vom Labormaßstab auf die großtechnische Produktion war nicht durchführbar.

Nach der Lehre der DE-OS 41 31 678 bzw. WO92/18227 können stabile multiple Emulsionen, insbesondere W/O/W-Emulsionen erhalten werden, wenn Emulgatorgemische verwendet werden, die aus mindestens je einem hydrophoben und einem hydrophilen Emulgator bestehen.

In diesen Schriften werden Verfahren zur Herstellung stabiler multipler Emulsionen beschrieben, dadurch gekennzeichnet, daß die hydrophilen Emulgatoren der Wasserphase und die hydrophoben Emulgatoren der Ölphase einverleibt werden und beide Phasen dann miteinander vereinigt werden.

Bei Befolgung dieser Lehre ist es zwar möglich, auf relativ einfache Weise zu kosmetisch eleganten multiplen Emulsionen, insbesondere zu stabilen W/O/W-Emulsionen zu gelangen, dennoch wären auch hier Verbesserungen wünschenswert.

Der Stand der Technik kennt nur solche Eintopfverfahren zur Herstellung multipler Emulsionen, welche unter Verwendung spezieller Emulgatorpaare hergestellt werden, bei denen der lipophile Emulgator in die Fettphase, der hydrophile Emulgator in die Wasserphase eingearbeitet wird. Unbekannt waren bisher Eintopfverfahren, nach denen unter Verwendung eines einzigen Emulgators stabile Emulsionen des Typs W/O/W erhältlich sind.

Es ist bekannt, daß hydrophile Emulgatoren bei steigender Temperatur ihr Löslichkeitsverhalten von wasserlöslich zu fettlöslich ändern. Die Temperatur, bei der die Emulgatoren ihre Löslichkeit ändern, wird Phaseninversionstemperatur (PIT) genannt.

S.Matsumoto (Journal of Colloid and Interface Science, Vol. 94, No.2, 1983) beschreibt, daß die Entwicklung einer W/O/W-Emulsion einer Phaseninversion konzentrierter W/O-Emulsionen, welche durch Span 80, einen ausgeprägten W/O-Emulgator, stabilisiert sind, vorausgeht. Matsumoto geht dabei von einem extrem unpolaren Öl, nämlich flüssigem Paraffin aus. Darüberhinaus sei eine gewisse Menge hydrophiler Emulgatoren zur Entwicklung einer W/O/W-Emulsion aus einer W/O-Emulsion nötig.

T.J.Lin, H.Kurihara und H.Ohta (Journal of the Society of Cosmetic Chemists 26, S. 121 - 139, März 1975 zeigen bei unpolaren Ölen, daß im Bereich der PIT extrem instabile multiple Emulsionen vorliegen können.

Aufgabe der vorliegenden Erfindung war also, stabile multiple Emulsionen zur Verfügung zu stellen und die Nachteile der Zubereitungen des Standes der Technik zu beseitigen.

Eine weitere Aufgabe der vorliegenden Erfindung war also, diesen Mangel des Standes der Technik zu beseitigen, und Verfahren zu entwickeln, welche selbst unter Verwendung eines einzigen Emulgators zu stabilen multiplen Emulsionen mit hoher Multiplizität führen.

Weiterhin war es die Aufgabe der Erfindung, Verfahren zu entwickeln, die es in einfacher Weise ermöglichen, gezielt multiple Emulsionen mit den erstrebten vorteilhaften Eigenschaften herzustellen.

Schließlich war es die Aufgabe der Erfindung, solche Verfahren zu entwickeln, welche auch im großtechnischen Maßstab zu reproduzierbar stabilen W/O/W-Emulsionen führen würde.

Es hat sich überraschenderweise gezeigt, und darin liegt die Lösung der Aufgaben begründet, daß
multiple Emulsionen vom Typ W/O/W, bestehend aus einer kontinuierlichen äußeren Wasserphase, einer darin dispergierten Ölphase und einer in dieser Ölphase dispergierten zweiten (inneren) Wasserphase, enthaltend

(a) mindestens einem Emulgator (Emulgator A), gewählt aus der Gruppe der Emulgatoren, deren Lipophilie mit

steigender Temperatur zunimmt und deren Hydrophilie mit sinkender Temperatur zunimmt, wobei der oder die Emulgatoren im Temperaturbereich von 40 - 90° C von einem HLB-Wert < 10 zu einem HLB-Wert > 10 wechseln, wobei der HLB-Wert des oder der Emulgatoren bei Raumtemperatur zwischen 11 und 18, bevorzugt zwischen 13 und 14, liegt und wobei der Emulgator bei der Vereinigung der Öl- mit der Wasserphase nicht vollständig in der Ölphase löslich ist

(b) eine Ölphase, wobei die Grundbestandteile der Ölphase gewählt werden aus der Gruppe der physiologisch verträglichen Öle, Fette und Wachse, welche für sich und/oder in Kombination einen "Erforderlichen HLB-Wert" (EHLB) von 10 - 20 oder aufweisen,
und/oder wobei
die Grundbestandteile der Ölphase gewählt werden aus der Gruppe der physiologisch verträglichen polaren Öle, Fette und Wachse, wobei die Polarität des oder der Grundbestandteile einzeln oder in Kombination kleiner als (ermittelt in Einheiten der Oberflächenspannung) 30 mN/m ist.

(d) eine äußere und eine innere Wasserphase, wobei diese Wasserphasen 0,1 - 5 Gew.-% (bezogen auf die Gesamtzusammensetzung) an organischen und/oder anorganischen Elektrolyten mit ein-, zwei- oder dreiwertigen Kationen enthalten,

(e) gewünschtenfalls weitere Hilfs- und/oder Zusatzstoffe, deren vorrangiger Zweck ist, die multiplen Emulsionströpfchen nach deren in-situ-Bildung zu stabilisieren, welche in die Ölphase und/oder die Wasserphasen eingearbeitet werden können,

(f) gewünschtenfalls weitere in der Kosmetik oder medizinischen Galenik übliche Grund-, Hilfs-, Zusatz-, und/oder Wirkstoffe, welche in die Ölphase und/oder die Wasserphasen eingearbeitet werden können,

Vorteilhafte Emulgatoren A stellen dabei Gemische aus Lecithin, Fettalkoholen und Fettsäuren dar, wie sie beispielsweise unter dem Handelsnamen Biophilic S erhältlich sind.

Sucrose-fettsäureester mit den entsprechenden Eigenschaften sind ebenfalls vorteilhafte Emulgatoren A, insbesondere Sucroselaurat, beispielsweise unter dem Handelsnamen Grilloten K87 erhältlich.

Weitere vorteilhafte Emulgatoren werden gewählt aus der Gruppe der Polyglycerin-monofettsäureester. Als günstige Vertreter dieser Substanzgruppe haben sich Polyglycerin-Isostearat, beispielsweise unter dem Handelsnamen Polydermanol GE-14 erhältlich, sowie Decaglyceryl-monoisostearat, beispielsweise erhältlich unter der Bezeichnung Decaglyn 1-IS, herausgestellt.

Ein Dimethicon-Copolyol, wie es unter der Bezeichnung DC 193 erhältlich ist, ist ebenfalls vorteilhaft zu verwenden.

Eine besonders vorteilhafte Verkörperung der vorliegenden Erfindung besteht in multiplen Emulsionen vom Typ W/O/W, bestehend aus einer kontinuierlichen äußeren Wasserphase, einer darin dispergierten Ölphase und einer in dieser Ölphase dispergierten zweiten (inneren) Wasserphase, enthaltend

(a) mindestens einem Emulgator der allgemeinen Formel

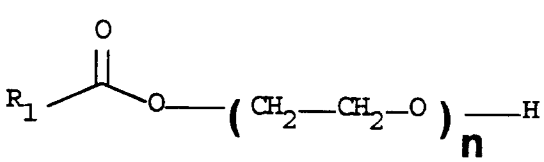

(Emulgator A),

wobei

$R^1 = C_{10-30}$ -Alkyl

n = eine Zahl von 8 bis 200

darstellen,

(b) eine Ölphase, wobei die Grundbestandteile der Ölphase gewählt werden aus der Gruppe der physiologisch verträglichen Öle, Fette und Wachse, welche für sich und/oder in Kombination einen Erforderlichen HLB-Wert (EHLB)

von 10 - 20 oder aufweisen,
und/oder wobei
die Grundbestandteile der Ölphase gewählt werden aus der Gruppe der physiologisch verträglichen polaren Öle, Fette und Wachse, wobei die Polarität des oder der Grundbestandteile einzeln oder in Kombination kleiner als 30 mN/m ist.

(d) eine äußere und eine innere Wasserphase, wobei diese Wasserphasen 0,1 - 5 Gew.-% (bezogen auf die Gesamtzusammensetzung) an organischen und/oder anorganischen Elektrolyten mit ein-, zwei- oder dreiwertigen Kationen enthalten,

(e) gewünschtenfalls weitere Hilfs- und/oder Zusatzstoffe, deren vorrangiger Zweck ist, die multiplen Emulsionströpfchen nach deren Bildung zu stabilisieren, welche in die Ölphase und/oder die Wasserphasen eingearbeitet werden können,

(f) gewünschtenfalls weitere in der Kosmetik oder medizinischen Galenik übliche Grund-, Hilfs-, Zusatz-, und/oder Wirkstoffe, welche in die Ölphase und/oder die Wasserphasen eingearbeitet werden können.

Vorteilhafte Emulgatoren A sind z.B. ethoxylierte $C_{14-22}$-Fettsäuren mit einem Ethoxylierungsgrad n, welcher mindestens 8 beträgt.

Vorteilhaft sind beispielsweise PEG-30-Stearat, PEG-40-Stearat, PEG-50-Stearat und/oder PEG-100-Stearat, bei denen also n = 30, 40, 50 bzw. 100 und $R_1$ = Heptadecyl ist. Natürlich weiß der Fachmann, daß der Ethoxylierungsgrad n keinen diskreten Wert darstellt, sondern in den entsprechenden Produkten eine gewisse Bandbreite einnimmt. Besonders vorteilhaft sind Produkte mit einem hohen Gehalt an Emulgatoren bei welchen der mittlere Ethoxylierungsgrad n Werte von etwa 30 bis 40 annimmt.

Insbesondere vorteilhaft sind Emulgatoren A mit HLB-Werten, die mindestens 10 betragen.

Nach der oben dargelegten Formel II errechnen sich

| | |
|---|---|
| für PEG-30-Stearat: | ein HLB-Wert von etwa 16,5 |
| für PEG-40-Stearat: | ein HLB-Wert von etwa 17,2 |
| für PEG-50-Stearat: | ein HLB-Wert von etwa 17,7 |
| für PEG-100-Stearat: | ein HLB-Wert von etwa 18,7. |

Diese Werte sind kennzeichnend für ausgeprägt hydrophile Emulgatoren mit - unter Standardemulsionsbedingungen - guten O/W-Emulgatoreigenschaften.

Vorteilhaft wird der Emulgator A in Konzentrationen von 0,05 - 10,0 Gew.-% eingesetzt, vorzugsweise in Konzentrationen von 0,1 - 5,0 Gew.-%, besonders bevorzugt in Konzentrationen von 0,5 - 2,0 Gew.-%, jeweils bezogen auf das Gesamtgewicht der Zubereitungen.

Für zu emulgierende Substanzen wird häufig ein sogenannter "Erforderlicher HLB-Wert" (in dieser Patentanmeldung EHLB genannt) aufgestellt. Dies bedeutet, daß eine Substanz mit einem EHLB-Wert von beispielsweise 15 in Wasser emulgiert werden kann von einem Emulgator mit dem HLB-Wert 15. Tabelle 1 listet häufig emulgierte Substanzen auf, deren EHLB zwischen 10 und 20 liegt. Der EHLB-Bereich zwischen 10 und 20 wird erfindungsgemäß bevorzugt.

Tabelle 1

| | |
|---|---|
| Alkohol, Cetyl- | 15 - 16 |
| Alkohol, Decyl- | 15 |
| Alkohol, Hexadecyl- | 11 - 12 |
| Alkohol, Isodecyl- | 14 |
| Alkohol, Isohexadecyl- | 11 - 12 |
| Alkohol, Lauryl- | 14 |

| | |
|---|---|
| Alkohol, Oleyl- | 13 - 14 |
| Alkohol, Stearyl- | 15 - 16 |
| Alkohol, Tridecyl- | 14 |
| Benzoesäureethylester | 13 |
| Butylstearat | 11 |
| Carnaubawachs | 15 |
| Decylacetat | 11 |
| Diethylanilin | 14 |
| Ethylanilin | 13 |
| Fenchon | 13 |
| Glycerylmonostearat | 13 |
| Isopropylmyristat | 13 |
| Isopropyllanolat | 14 |
| Isopropylpalmitat | 11 - 12 |
| Kiefernadelöl | 16 |
| Laurinsäure | 16 |
| Maisöl | 10 |
| Linolsäure | 16 |
| Menhadenöl | 12 |
| Methylphenylsilicon | 11 |
| Mineralöl, naphthenisch (leicht) | 11 - 12 |
| Mineralöl, paraffinbasisch | 10 |
| Mineralöl, (leicht) | 10 - 11 |
| Ölsäure | 17 |
| Palmöl | 10 |
| PEG-30-Cetylether | 10 - 11 |
| Petroleum | 14 |
| Polyethylenwachs | 15 |
| Propylen, Tetramer | 14 |
| Rizinusöl | 14 |
| Ricinolsäure | 16 |
| Tafelparaffin | 10 |

Erfindungsgemäß besonders bevorzugt ist, den EHLB der Ölkomponente oder der Ölkomponenten aus dem Bereich von 10 bis 18, insbesondere von 13 bis 16 zu wählen. Ganz besonders

bevorzugt ist ein EHLB von etwa 14. Wahlweise, aber nicht notwendig alternativ, können die erforderlichen Eigenschaften der Grundbestandteile der Ölphase auch so festgelegt werden, daß bestimmte Polaritäten gewählt werden.

Öle und Fette unterscheiden sich unter anderem in ihrer Polarität, welche schwierig zu definieren ist. Es wurde

bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Erfindungsgemäß wird die Ölphase gewählt wird aus der Gruppe der polaren Ölkomponenten, welche eine Polarität zwischen 10 und 30 mN/m aufweisen.

Vorteilhafte Ausführungsformen der vorliegenden Erfindung werden erhalten, wenn die Ölphase gewählt wird aus der Gruppe der polaren Ölkomponenten, besonders bevorzugt aus der Gruppe der Ölkomponenten, welche eine Polarität zwischen 10 und 20 mN/m aufweisen.

Es ist daher besonders vorteilhaft, wenn Ölkomponenten beide in den gesetzten Grenzen definierten Bedingungen erfüllen, wie für EHLB und für Polarität oben beschrieben. Gleichwohl können die vorteilhaften Ölkomponenten im Einzelfalle Zahlenwerte für EHLB und Polarität annehmen, dergestalt daß der Wert für das eine Phänomen im erfindungsgemäß beanspruchten Bereich liegt, der Wert für das andere Phänomen aber nicht.

Der erfindungsgemäße Vorteil, polare Öle zu verwenden, liegt darin, daß diese Öle die Bildung der W/O/W-Emulsion begünstigen.

Vorteilhaft ist, polare pflanzliche Öle als Hauptkomponente der Ölphase zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl, Kukuinußöl, Safloröl, Distelöl, Nachtkerzenöl und weiteren Ölen, welche mindestens 1,5 Gew.-% an Linolsäureglyceriden enthalten.

Es ist ferner vorteilhaft, außer dem Emulgator oder den Emulgatoren A einen oder mehrere Stabilisatoren der allgemeinen Formel

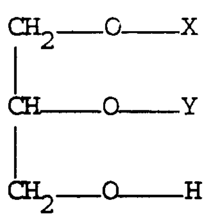

(Stabilisator 1),

einzusetzen, wobei
einer der Reste X oder Y ein Wasserstoffatom darstellt und der verbleibende Rest Y oder X gewählt wird aus der Gruppe der verzweigten oder unverzweigten Acylreste mit 10 - 30 Kohlenstoffatomen.

Stabilisator 1 ist als lipophiler Gerüstbildner anzusehen. Bevorzugt wird als Stabilisator 1 das Glycerinmonostearat gewählt.

Weiterhin ist von erheblichem Vorteil, der Ölphase einen oder mehrere verzweigte und/oder unverzweigte aliphatische Fettalkohole und/oder Fettsäuren mit 8 bis 18 Kohlenstoffatomen, insbesondere mit 16 Kohlenstoffatomen, namentlich Cetylalkohol, einzuverleiben.

Die unverzweigten aliphatischen Fettalkohole und/oder Fettsäuren mit 8 bis 18 Kohlenstoffatomen (Stabilisator 2) tragen ebenfalls nicht oder nicht in wesentlichem Maße zur Bildung der multiplen Emulsionströpfchen bei, stabilisieren makroskopisch aber bereits gebildete multiple Emulsionströpfchen.

Als weiterer fakultativer Stabilisator 3 können vorteilhaft hydrophile Gerüstbildner verwendet werden.

Hydrophile Gerüstbildner sind Acrylate, aber auch natürliche, synthetische bzw. partialsynthetische Polysaccharide bzw. Proteine oder deren Derivate, beispielsweise Xanthangummi oder Hydroxypropylguargummi bzw. Kollagen usw. Erfindungsgemäß tragen der oder die Stabilisatoren 1, 2 bzw. 3 nicht oder nicht in wesentlichem Maße zur Bildung der multiplen Emulsionströpfchen bei, stabilisieren aber bereits gebildete multiple Emulsionströpfchen.

Zwar können die Stabilisatoren 1, 2 und 3 sowohl in beliebiger Kombination miteinander, aber auch jeweils alleine in den erfindungsgemäßen W/O/W-Emulsionen verwendet werden.

Wenn diese Stabilisatoren jedoch einzeln oder als Zweierkombinationen verwendet werden, so gibt die Bezifferung

an, daß Stabilisator 1 der bevorzugte gegenüber den Stabilisatoren 2 und 3 ist, der Stabilisator 2 der bevorzugte gegenüber Stabilisator 3 ist.

Es war erstaunlich und nicht vorhersehbar, daß bei der Verwendung eines einzigen Emulgators (nämlich des Emulgators A), welcher darüberhinaus ausgeprägte O/W-Emulgatoreigenschaften trägt, der in der Ölphase, und nur in dieser eingesetzt wird, stabile multiple Emulsionen mit äußerst vorteilhaften Eigenschaften erhalten werden können.

Weiter war nicht vorhersehbar, daß unter den erfindungsgemäßen Bedingungen stabile W/O/W-Emulsionen in Eintopfverfahren herstellbar sind.

Eine Homogenisierung der Emulsion nach Phasenvereinigung zwischen 40 und 80° C steuert überraschenderweise die Tröpfchengröße der multiplen Tropfchen, ohne aber die Multiplizität des Systems zu beeinflussen.

Schließlich war erstaunlich, daß unter der (an sich fakultativen) Verwendung von Stabilisatoren der Typen 1, 2 bzw. 3, diese ebenfalls in der Ölphase, und nur in dieser, eingesetzt, eine erhebliche Steigerung der Stabilität der erfindungsgemäß erhaltenen W/O/W-Emulsionen möglich ist.

Günstig werden der oder die Stabilisatoren 1, 2 bzw. 3 in Konzentrationen von 0,05 - 15,0 Gew.-% eingesetzt, vorzugsweise in Konzentrationen von 0,1 - 7,5 Gew.-%, besonders bevorzugt in Konzentrationen von 0,5 - 5,0 Gew.-%, jeweils bezogen auf das Gesamtgewicht der Zubereitungen.

Ferner ist es von Vorteil, hydrophile und/oder lipophile Gelbildner einzusetzen. Diese tragen zwar in der Regel nicht zur Bildung multipler Tröpfchen bei, förden aber die Stabilität einmal gebildeter multipler Tröpfchen.

Der Zusatz von Elektrolyten bewirkt eine Veränderung des Löslichkeitsverhaltens eines hydrophilen Emulgators an der PIT, wenn er in einer polaren Ölphase mit den vorab beschriebenen Eigenschaften eingesetzt wird. Die hydrophilen Emulgatoren mit den vorab beschriebenen Strukturen bzw. Eigenschaften durchlaufen eine partielle Phaseninversion, in der es zu einer Solubilisierung von Wasser durch die polare Ölphase kommt, welche in einer mikroskopisch stabilen W/O/W-Emulsion resultiert.

Die erfindungsgemäßen W/O/W-Emulsionen enthalten daher Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen W/O/W-Emulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Besonders vorteilhaft ist ferner, wenn die Ölphase keine Komponenten enthält, die einen Schmelzpunkt unter 40° C haben.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen

Erfindungsgemäß enthalten die W/O/W-Emulsionen vorteilhaft ein oder mehrere Antioxidantien. Als günstige, aber dennoch fakultativ zu verwendende Antioxidantien alle für kosmetische und/oder dermatologische Anwendungen geeigneten oder gebräuchlichen natürlichen, synthetischen und/oder partialsynthetischen Antioxiantien verwendet werden.

Besonders vorteilhaft werden die Antioxidantien gewählt aus der Gruppe bestehend aus

Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$ - Carotin, $\beta$ - Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin

und deren Glycosyl-, N-Acetyl-, Methyl-, Ethyl-, Propyl-, Amyl-, Butyl- und Lauryl-, Palmitoyl-, Oleyl-, γ - Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α - Hydroxyfettsäuren, Palmitinsäure, Phytinsäure, Lactoferrin), α - Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ - Linolensäure, Linolsäure, Ölsäure), Folsäure und deren Derivate, Ubiquinon und Ubiquinol deren Derivate, Vitamin C und Derivate (z.B. Ascorbylpalmitate, Mg - Ascorbylphosphate, Ascorbylacetate), Tocopherole und Derivate (z .B. Vitamin- E - acetat), Vitamin A und Derivate (z.B. Vitamin A - palmitat) sowie Konyferylbenzoat des Benzoeharzes, Flavonen oder Flavonoiden, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Es ist ferner von Vorteil, den erfindungsgemäßen W/O/W-Emulsionen Vitamine, Enzyme, Coenzyme, insbesondere Biotin bzw. Biotinester, aber auch andere in der Kosmetik und Dermatologie übliche Substanzen dieser oder verwandter Art, beispielsweise Aktivatoren wie Citronensäure, zuzufügen.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper sowie Wasser.

Es ist bei Befolgung der erfindungsgemäßen Lehre möglich, das Verhältnis des Gesamtgewichts der der Ölphase zu dem Gesamtgewicht der Summe der beiden wäßrigen Phasen im Intervall von 10 : 90 bis 50 : 50 einzustellen.

Durch geeignete Puffer ist es möglich, jeden für kosmetische oder dermatologische Zwecke akzeptablen pH-Wert einzustellen, ohne daß die Stabilität der erfindungsgemäßen Emulsionen darunter leiden würde, beispielsweise im pH-Intervall von 3 - 8,5.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,

Derivate des Benzophenons, vorzugsweise 2-Hydroxy-4-methoxybenzophenon, 2 -Hydroxy-4 -methoxy-4'-methylbenzophenon, 2,2'-Dihydroxy-4-methoxybenzophenon;

- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäßen Emulsionen UVA-Filtersubstanzen, können diese erfindungsgemäß vorteilhaft

gewählt werden aus der Gruppe der Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion.

Die erfindungsgemäßen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Vorteilhaft sind auch solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter und/oder mindestens ein anorganisches Pigment.

Ferner sind gerade solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern und/oder dem oder den anorganischens Pigmenten ein oder mehrere Antioxidantien enthalten.

Die erfindungsgemäßen W/O/W-Emulsionen können grundsätzlich alle kosmetischen Verwendungszwecke erfüllen, die üblicherweise Emulsionen zu erfüllen haben, beispielsweise Tagesrèmes, Nachtcrèmes, Hand- oder Körpercrèmes, Sonnenschutzformulierungen, Nährstoffcrèmes, Liposomencrèmes, Vitamincrèmes usw.

Es ist dabei sowohl vorteilhaft, die erfindungsgemäßen W/O/W-Emulsionen auf dem Gebiete der pflegenden Kosmetik als auch auf dem Gebiete der dekorativen Kosmetik zu verwenden.

Es ist aber gegebenenfalls auch vorteilhaft, erfindungsgemäße Emulsionen auch als Trägersubstanz dermatologischer oder topischer Zubereitungen zu verwenden.

Erfindungsgemäß ist ferner ein Verfahren zur Herstellung von W/O/W-Emulsionen, dadurch gekennzeichnet, daß man

Wasser, enthaltend 0,1 - 5 Gew.-% (bezogen auf die Gesamtzusammensetzung) an organischen und/oder anorganischen Elektrolyten mit ein-, zwei- und/oder dreiwertigen Kationen,

und eine Ölphase, diese Ölphase enthaltend

(a) mindestens einem Emulgator (Emulgator A), gewählt aus der Gruppe der Emulgatoren, deren Lipophilie mit steigender Temperatur zunimmt und deren Hydrophilie mit sinkender Temperatur zunimmt, wobei der oder die Emulgatoren im Temperaturbereich von 40 - 90° C von einem HLB-Wert < 10 zu einem HLB-Wert > 10 wechseln, wobei der HLB-Wert des oder der Emulgatoren bei Raumtemperatur zwischen 11 und 18, insbesondere 13 und 14 liegt und wobei der Emulgator bei Vereinigung der Öl- mit der Wasserphase nicht vollständig in der Ölphase löslich ist,
insbesondere mindestens einem Emulgator der allgemeinen Formel

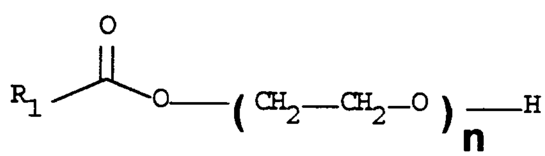

(Emulgator A),

wobei

$R^1 = C_{10-30}$-Alkyl
n = eine Zahl von 8 bis 200

darstellen,

(b) gewünschtenfalls ferner enthaltend:

(b1) einen oder mehrere Stabilistoren der allgemeinen Formel

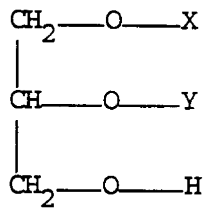

(Stabilisator 1),

einzusetzen, wobei
einer der Reste X oder Y ein Wasserstoffatom darstellt und der verbleibende Rest Y oder X gewählt wird aus der Gruppe der verzweigten oder unverzweigten Acylreste mit 10 - 30 Kohlenstoffatomen und oder
(b2) einen oder mehrere verzweigte und/oder unverzweigte aliphatische Fettalkohole mit 8 bis 18 Kohlenstoffatomen, insbesondere mit 16 Kohlenstoffatomen, namentlich Cetylalkohol,

(c) die Grundkomponenten dieser Ölphase, wobei
die Grundbestandteile der Ölphase gewählt werden aus der Gruppe der physiologisch verträglichen Öle, Fette und Wachse, welche für sich und/oder in Kombination einen Erforderlichen HLB-Wert (EHLB) von 10 - 20 oder aufweisen,
und/oder wobei
die Grundbestandteile der Ölphase gewählt werden aus der Gruppe der physiologisch verträglichen polaren Öle, Fette und Wachse, wobei die Polarität des oder der Grundbestandteile einzeln oder in Kombination kleiner als 30 mN/m ist,
bei einer Temperatur zusammengibt, bei welcher die Ölphase und die Wasserphase im wesentlichen in flüssiger Form vorliegen und die so entstandene Mischung unter ständiger Agitation hält, gegebenenfalls einem oder mehreren Homogenisierungsschritten unterwirft und die so entstandene W/O/W-Emulsion hernach auf Raumtemperatur abkühlen läßt.

Ganz besonders vorteilhaft ist ein erfindungsgemäßes Verfahren, bei dem die W/O/W-Emulsion bei einer Temperatur hergestellt wird, die oberhalb der Phaseninversionstemperatur (PIT) liegt, und hernach auf eine Temperatur gebracht wird, welche unterhalb der PIT liegt.

Dabei liegt im allgemeinen Fachwissen des Fachmannes und bedarf keinerlei erfinderischen Dazutuns, für einen gegebenen Emulgator oder ein gegebenes Emulgatorsystem in einem gegebenen Wasser/Ölphasensystem die PIT zu ermitteln.

Als allgemeiner Richtwert für die PIT bei üblichen Emulgatorkonzentrationen kann ein Temperaturbereich von etwa 40 - 90° C angegeben werden. Im allgemeinen sinkt die PIT bei steigender Emulgatorkonzentration.

Es können während dieses Verfahrens gewünschtenfalls ferner die in der Kosmetik oder medizinischen Galenik üblichen Grund-, Hilfs-, Zusatz-, und/oder Wirkstoffe zugegeben werden. Es ist dem Fachmann klar, zu welchem Zeitpunkt solche Stoffe dem Prozeß beigegeben werden können, ohne daß die Eigenschaften der zu erzielenden W/O/W-Emulsion wesentlich beeinträchtigt werden.

Beispielsweise, und dies kann sich gegebenenfalls als besonders vorteilhaft erweisen, kann die externe Wasserphase eine andere Zusammensetzung erhalten als die innere, indem nachträglich, in die bereits gebildete W/O/W-Emulsion, wasserlösliche Stoffe, auch Öle, eingebracht werden. Werden die dem Fachmanne bekannten Rahmenbedingungen eingehalten, erfolgt dabei keine Änderung der Stabilität bzw. Struktur der

Zwar wird in WO 93/11865 ein Verfahren zur Herstellung von O/W-Emulsionen von polaren Ölkörpern beschrieben, dadurch gekennzeichnet, daß man einen polaren Ölkörper mit einem nichtionischen Emulgator (mit einem HLB-Wert von 10 bis 18), einem Grenzflächen-Moderator (gewählt aus der Gruppe der Tocopherole und anderer Substanzen) und gegebenenfalls mit einem Co-Emulgator aus der Gruppe der Fettalkohole oder der Partialester von Polyolen mit Fettsäuren in Gegenwart von 8 bis 85 Gew.-% Wasser bei einer Temperatur oberhalb des Schmelzpunktes des Gemisches aus den Komponenten emulgiert und die Emulsion auf eine Temperatur oberhalb der PIT erhitzt oder die Emulsion bei dieser Temperatur herstellt und dann die Emulsion auf eine Temperatur unterhalb der PIT abkühlt.

Mit dem in WO 93/11865 beschriebenen Verfahren werden aber eben nur O/W-Emulsionen erhalten.

Unter Verwendung der erfindungsgemäßen Komponenten tritt bei dem erfindungsgemäßen Verfahren keine vollständige Phaseninversion auf, sondern eine partielle. Das Endprodukt stellen multilamellare Phasen stabilisierte W/O/W-Tröpfchen dar.

Werden erfindungsgemäße W/O/W-Emulsionen gemäß dem in der WO 93/11865 beschriebenen Verfahren nach

Entstehen der multiplen Tröpfchen auf eine Temperatur erhitzt wird, die oberhalb der PIT liegt, wandeln sie sich in O/W-Emulsionen um.

Die nachfolgenden Beispiele betreffen vorteilhafte erfindungsgemäße Ausführungsformen. Die Zahlenwerte bedeuten stets Gewichtsprozente, bezogen auf das Gesamtgewicht der Zubereitungen, sofern nicht ausdrücklich etwas Anderes angemerkt ist.

Beispiel 1

| | |
|---|---|
| PEG-100-Stearat | 2,00 % |
| Glycerylstearat | 4,00 % |
| Squalan | 1,50 % |
| Squalen | 1,50 % |
| Isopropylpalmitat | 5,40 % |
| Magnesiumsulfat | 0,60 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 2

| | |
|---|---|
| PEG-40-Stearat | 1,00 % |
| Glycerylstearat | 2,00 % |
| Cetylalkohol | 3,00 % |
| Mineralöl DAB 9 | 2,00 % |
| Safloröl | 2,00 % |
| Isopropylpalmitat | 4,50 % |
| Glycerin | 3,00 % |
| Magnesiumsulfat | 1,20 % |
| Konserv.-Mittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 3

| | |
|---|---|
| PEG-80-Stearat | 2,00 % |
| Cetylalkohol | 3,00 % |
| Mineralöl DAB 9 | 1,50 % |
| Nachtkerzenöl | 2,50 % |
| Isopropylpalmitat | 5,40 % |
| Propylenglycol | 3,00 % |
| Kaliumchlorid | 0,60 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 4

| | |
|---|---|
| Steareth-100 | 2,00 % |
| Myristylalkohol | 1,00 % |
| Mineralöl DAB 9 | 3,00 % |
| Rizinusöl | 3,00 % |
| Cyclomethicone | 2,00 % |
| Propylenglycol | 3,00 % |
| Glycerin | 5,00 % |
| Kaliumchlorid | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 5

| | |
|---|---|
| Steareth-20 | 2,00 % |
| Cetearyl | 3,00 % |
| Vaseline | 0,50 % |
| Weizenkeimöl | 1,50 % |
| Dimethicone | 5,00 % |
| Glycerin | 5,00 % |
| Natriumchlorid | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 6

| | |
|---|---|
| Dimethicon Copolyol | 2,00 % |
| Cetearylalkohol | 3,00 % |
| Vaseline | 0,50 % |
| Weizenkeimöl | 1,50 % |
| Dimethicone | 5,00 % |
| Glycerin | 5,00 % |
| Natriumchlorid | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 7

| | |
|---|---|
| PEG-20-Behenat | 2,00 % |
| Stearylalkohol | 3,00 % |
| Vaseline | 1,00 % |
| Traubenkernöl | 3,00 % |
| Dimethicone | 3,00 % |
| Sorbit | 5,00 % |
| Zinksulfat | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 8

| | |
|---|---|
| Decaglyn 1-IS | 2,00 % |
| Stearylalkohol | 3,00 % |
| Vaseline | 1,00 % |
| Traubenkernöl | 3,00 % |
| Dimethicone | 3,00 % |
| Sorbit | 5,00 % |
| Zinksulfat | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 9

| | |
|---|---|
| PEG-20-Myristat | 2,00 % |
| Stearylalkohol | 3,00 % |
| Vaseline | 2,00 % |
| Rizinusöl | 5,00 % |
| Dimethicone | 5,00 % |
| Sorbit | 5,00 % |
| Zinksulfat | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 10

| | |
|---|---|
| Sucroselaurat | 2,00 % |
| Stearylalkohol | 3,00 % |
| Vaseline | 2,00 % |
| Rizinusöl | 5,00 % |
| Dimethicone | 5,00 % |
| Sorbit | 5,00 % |
| Zinksulfat | 3,00 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

Beispiel 11

| | |
|---|---|
| PEG-80-Behenat | 2,00 % |
| Glycerylbehenat | 4,00 % |
| Squalan | 3,00 % |
| Rizinusöl | 5,40 % |
| Glycerin | 6,00 % |
| Magnesiumsulfat | 2,60 % |
| Konservierungsmittel | 0,50 % |
| Wasser VES | ad 100,00 % |

Die Fettphase, welche den Emulgator enthält, wird auf 80° C erhitzt, die Wasserphase ebenfalls. Bei 80° C werden beide Phasen miteinander vereinigt, ca. 3 - 10 Minuten lang homogenisiert und dann auf Raumtemperatur abgekühlt.

**Patentansprüche**

**1.** Multiple Emulsionen vom Typ W/O/W, bestehend aus einer kontinuierlichen äußeren Wasserphase, einer darin dispergierten Ölphase und einer in dieser Ölphase dispergierten zweiten (inneren) Wasserphase, enthaltend

(a) mindestens einem Emulgator (Emulgator A), gewählt aus der Gruppe der Emulgatoren, deren Lipophilie mit steigender Temperatur zunimmt und deren Hydrophilie mit sinkender Temperatur zunimmt, wobei der oder die Emulgatoren im Temperaturbereich von 40 - 90° C von einem HLB-Wert < 10 zu einem HLB-Wert > 10 wechseln, wobei der HLB-Wert des oder der Emulgatoren bei Raumtemperatur zwischen 11 und 18, bevorzugt zwischen 13 und 14, liegt und wobei der Emulgator bei der Vereinigung der Öl- mit der Wasserphase nicht vollständig in der Ölphase löslich ist
(b) eine Ölphase, wobei die Grundbestandteile der Ölphase gewählt werden aus der Gruppe der physiologisch verträglichen Öle, Fette und Wachse, wobei

- der einzige Grundbestandteils der Ölphase, falls diese nur eine einzige Ölkomponente enthält oder
- die Kombination der Grundbestandteile der Ölphase, sofern die Ölphase mehrere Ölkomponenten enthält

einen "Erforderlichen HLB-Wert" (EHLB) von 10 - 20 und/oder eine Polarität kleiner als (ermittelt in Einheiten der Oberflächenspannung) 30 mN/m aufweist.
(c) eine äußere und eine innere Wasserphase, wobei diese Wasserphasen 0,1 - 5 Gew.-% (bezogen auf die Gesamtzusammensetzung) an organischen und/oder anorganischen Elektrolyten mit ein-, zwei- oder dreiwertigen Kationen enthalten,
(d) gewünschtenfalls weitere Hilfs- und/oder Zusatzstoffe, deren vorrangiger Zweck ist, die multiplen Emulsionströpfchen nach deren in-situ-Bildung zu stabilisieren, welche in die Ölphase und/oder die Wasserphasen eingearbeitet werden können,
(e) gewünschtenfalls weitere in der Kosmetik oder medizinischen Galenik übliche Grund-, Hilfs-, Zusatz-, und/oder Wirkstoffe, welche in die Ölphase und/oder die Wasserphasen eingearbeitet werden können,

**2.** Emulsionen nach Anspruch 1, dadurch gekennzeichnet, daß der oder die Emulgatoren A gewählt werden aus der Gruppe der Emulgatoren der allgemeinen Formel

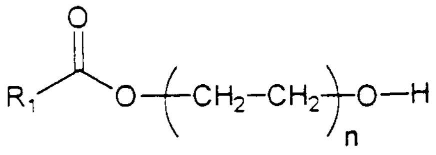 (Emulgator A),

wobei

$R_1 = C_{10-30}$-Alkyl

n = eine Zahl von 8 bis 200

darstellen.

3. Emulsionen nach Anspruch 2, dadurch gekennzeichnet, daß der oder die Emulgatoren A gewählt werden aus der Gruppe bei welchen n Werte von 20 bis 40 annimmt.

4. Emulsionen nach Anspruch 1, dadurch gekennzeichnet daß der oder die Emulgatoren A gewählt werden aus der Gruppe

(a) der Gemische aus Lecithin, Fettalkoholen und Fettsäuren dar, wie sie beispielsweise unter dem Handelsnamen Biophilic®S erhältlich sind,
(b) Sucroselaurat, wie beispielsweise unter dem Handelsnamen Grilloten® K87 erhältlich,
(c) der Polyglycerin-monofettsäureester, beispielsweise Polyglycerin-Isostearat wie unter dem Handelsnamen Polydermanol® GE-14 erhältlich, sowie Decaglyceryl-monoisostearat wie unter der Bezeichnung Decaglyn® 1-IS erhältlich,
(d) Dimethicon-Copolyole wie unter der Bezeichnung DC 193 erhältlich.

5. Emulsionen nach Anspruch 1, enthaltend einen oder mehrere Stabilisatoren der allgemeinen Formel (1)

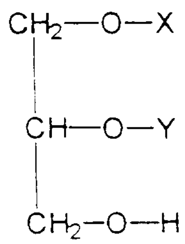

wobei einer der Reste X oder Y ein Wasserstoffatom darstellt und der verbleibende Rest Y oder X gewählt wird aus der Gruppe der verzweigten oder unverzweigten Acylreste mit 10 - 30 Kohlenstoffatomen.

6. Emulsionen nach Anspruch 5, dadurch gekennzeichnet, daß als Stabilisator 1 das Glycerinmonostearat gewählt wird.

7. Emulsionen nach Anspruch 6, dadurch gekennzeichnet, daß der oder die Emulgatoren A und/oder der oder die Stabilisatoren 1 in Konzentrationen von 0,05 - 15,0 Gew.-%, vorzugsweise in Konzentrationen von 0,1 - 7,5 Gew.-%, besonders bevorzugt in Konzentrationen von 0,5 - 5,0 Gew.-% eingesetzt werden, jeweils bezogen auf das Gesamtgewicht der Zubereitungen.

8. Emulsionen nach Anspruch 1, dadurch gekennzeichnet, daß die Ölphase gewählt wird aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl Kukuinußöl, Safloröl, Diestelöl, Nachtkerzenöl und werteren Ölen, welche mindestens 1,5 Gew.-% an Linolsäureglyceriden enthalten.

**9.** Emulsionen nach Anspruch 1, dadurch gekennzeichnet, daß die Ölphase gewählt wird aus der Gruppe der Ölkomponenten, welche eine Polarität zwischen 10 und 30 mN/m aufweisen.

**10.** Emulsionen nach Anspruch 1, dadurch gekennzeichnet, daß die Ölkomponenten gewählt werden aus der Gruppe der Öle, welche eine Polarität zwischen 10 und 20 mN/m aufweisen.

**11.** Emulsionen nach Anspruch 1, dadurch gekennzeichnet, daß der Ölphase ein oder mehrere verzweigte und/oder unverzweigte aliphatische Fettalkohole und/oder Fettsäuren mit 8 bis 18 Kohlenstoffatomen einverleibt werden.

**12.** Emulsionen nach Anspruch 1, dadurch gekennzeichnet, daß die Ölphase keine Komponenten enthält, die einen Schmelzpunkt unter 40° C haben.

**13.** Emulsionen nach Anspruch 1, dadurch gekennzeichnet, daß der Wasserphase Elektrolyte, insbesondere Kochsalz zugesetzt werden, vorteilhaft in Konzentrationen von 0,1 - 5,0 Gew.-%, besonders vorteilhaft etwa 0,6 3.0 Gew.%, bezogen auf das Gesamtgewicht der Zubereitung.

**14.** Verfahren zur Herstellung von W/O/W-Emulsionen, dadurch gekennzeichnet, daß man Wasser, enthaltend 0,1 - 5 Gew.-% (bezogen auf die Gesamtzusammensetzung) an organischen und/oder anorganischen Elektrolyten mit ein- und/oder zweiwertigen Kationen, und eine Ölphase, diese Ölphase enthaltend

(a) mindestens einem Emulgator (Emulgator A), gewählt aus der Gruppe der Emulgatoren, deren Lipophilie mit steigender Temperatur zunimmt und deren Hydrophilie mit sinkender Temperatur zunimmt, wobei der oder die Emulgatoren im Temperaturbereich von 40 - 90° C von einem HLB-Wert < 10 zu einem HLB-Wert > 10 wechseln, wobei der HLB-Wert des oder der Emulgatoren bei Raumtemperatur zwischen 11 und 18, bevorzugt zwischen 13 und 14, liegt und wobei der Emulgator bei etwa 80° C nicht vollständig in der Ölphase löslich ist, insbesondere mindestens einem Emulgator der allgemeinen Formel

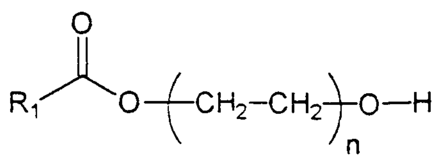

(Emulgator A),

wobei

$R_1 = C_{10-30}$ -Alkyl

n = eine Zahl von 8 bis 200

darstellen,

(b) gewünschtenfalls ferner enthaltend:

(b1) einen oder mehrere Stabilisatoren der allgemeinen Formel

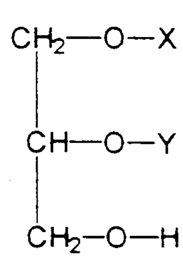

(Stabilisator 1),

wobei einer der Reste X oder Y ein Wasserstoffatom darstellt und der verbleibende Rest Y oder X gewählt

wird aus der Gruppe der verzweigten oder unverzweigten Acylreste mit 10 - 30 Kohlenstoffatomen und oder

(b2) einen oder mehrere verzweigte und/oder unverzweigte aliphatische Fettalkohole mit 8 bis 18 Kohlenstoffatomen, insbesondere mit 16 Kohlenstoffatomen, namentlich Cetylalkohol,

(c) die Grundkomponenten dieser Ölphase, welche gewählt werden aus der Gruppe der physiologisch verträglichen Öle, Fette und Wachse, wobei

- der einzige Grundbestandteil der Ölphase, falls diese nur eine einzige Ölkomponente enthält oder
- die Kombination der Grundbestandteile der Ölphase, sofern die Ölphase mehrere Ölkomponenten enthält

einen "Erforderlichen HLB-Wert" (EHLB) von 10 - 20 und/oder eine Polarität kleiner als (ermittelt in Einheiten der Oberflächenspannung) 30 mN/m aufweist,

bei einer Temperatur zusammengibt, bei welcher die Ölphase und die Wasserphase im wesentlichen in flüssiger Form vorliegen und die so entstandene Mischung unter ständiger Agitation hält, gegebenenfalls einem oder mehreren Homogenisierungsschritten unterwirft und die so entstandene W/O/W-Emulsion hernach auf Raumtemperatur abkühlen läßt.

**15.** Verfahren nach Anspruch 14, dadurch gekennzeichnet, daß die W/O/W-Emulsion bei einer Temperatur hergestellt wird, die oberhalb der PIT liegt und hernach auf eine Temperatur gebracht wird, welche unterhalb der PIT liegt.

## Claims

**1.** Multiple emulsions of the W/O/W type consisting of a continuous external aqueous phase, an oily phase dispersed therein and a second (internal) aqueous phase dispersed in this oily phase, comprising

(a) at least one emulsifier (emulsifier A), chosen from the group consisting of emulsifiers, the lipophilicity of which increases with increasing temperature and the hydrophilicity of which increases with decreasing temperature, the emulsifier or emulsifiers changing from an HLB value < 10 to an HLB value > 10 in the temperature range of 40 - 90°C, the HLB value of the emulsifier or emulsifiers at room temperature being between 11 and 18, preferably between 13 and 14, and the emulsifier not being completely soluble in the oily phase when the oily phase is combined with the aqueous phase,
(b) an oily phase, where the base constituents of the oily phase are chosen from the group consisting of physiologically tolerated oils, fats and waxes, where

- the single base constituent of the oily phase, if this comprises only a single oil component, or
- the combination of the base constituents of the oily phase, if the oily phase comprises several oil components

has a "required HLB value" (RHLB) of 10 - 20, and/or a polarity, (determined in units of surface tension) of less than 30 mN/m,
(c) an external and an internal aqueous phase, these aqueous phases comprising 0.1 - 5 % by weight (based on the total composition) of organic and/or inorganic electrolytes with mono-, di- or trivalent cations,
(d) if desired further auxiliaries and/or additives, the chief purpose of which is to stabilize the multiple emulsion droplets, after in situ formation thereof, and which can be incorporated into the oily phase and/or the aqueous phases,
(e) if desired other bases, auxiliaries, additives and/or active compounds customary in cosmetics or medical galenics, which can be incorporated into the oily phase and/or the aqueous phases.

**2.** Emulsions according to Claim 1, characterized in that the emulsifier or emulsifiers A are chosen from the group consisting of emulsifiers of the general formula

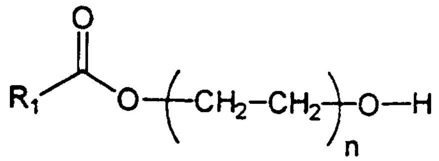 (emulsifier A),

in which

$R^1 = C_{10-30}$ -alkyl and
n = a number from 8 to 200.

3. Emulsions according to Claim 2, characterized in that the emulsifier or emulsifiers A are chosen from the group in which n assumes values of 20 to 40.

4. Emulsions according to Claim 1, characterized in that the emulsifier or emulsifiers A are chosen from the group consisting of

(a) mixtures of lecithin, fatty alcohols and fatty acids such as are obtainable, for example, under the trade name Biophilic® S,
(b) sucrose laureate, such as is obtainable, for example, under the trade name Grilloten® K87,
(c) polyglycerol mono-fatty acid esters, for example polyglycerolisostearate, such as is obtainable under the trade name Polydermanol® GE-14, and decaglyceryl monoisostearate, such as is obtainable under the name Decaglyn® 1-IS,
(d) dimethicone copolyols, such as are obtainable under the name DC 193.

5. Emulsions according to Claim 1, comprising one or more stabilizers of the general formula (1)

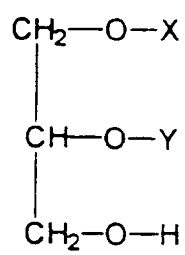

in which
one of the radicals X or Y is a hydrogen atom and the remaining radical Y or X is chosen from the group consisting of branched or unbranched acyl radicals having 10 - 30 carbon atoms.

6. Emulsions according to Claim 5, characterized in that glycerol monostearate is chosen as stabilizer 1.

7. Emulsions according to Claim 6, characterized in that the emulsifier or emulsifiers A and/or the stabilizer or stabilizers 1 are employed in concentrations of 0.05 - 15.0% by weight, preferably in concentrations of 0.1 - 7.5% by weight, particularly preferably in concentrations of 0.5 - 5.0% by weight, in each case based on the total weight of the formulations.

8. Emulsions according to Claim 1, characterized in that the oily phase is chosen from the group consisting of oils of the plant families Euphorbiaceae, Poaceae, Fabaceae, Brassicaceae, Pedalaceae, Asteraceae, Linaceae, Flacourticaceae and Violales, preferably chosen from the group of naturally occurring castor oil, wheatgerm oil, grapeseed oil, coconut oil, safflower oil, thistle oil, evening primrose oil and other oils which comprise at least 1.5 % by weight of linoleic acid glycerides.

9. Emulsions according to Claim 1, characterized in that the oily phase is chosen from the group consisting of oil components which have a polarity of between 10 and 30 mN/m.

10. Emulsions according to Claim 1, characterized in that the oil components are chosen from the group consisting of oils which have a polarity of between 10 and 20 mN/m.

11. Emulsions according to Claim 1, characterized in that one or more branched and/or unbranched aliphatic fatty alcohols and/or fatty acids having 8 to 18 carbon atoms are incorporated into the oily phase.

12. Emulsions according to Claim 1, characterized in that the oily phase comprises no components which have a melting point below 40°C.

13. Emulsions according to Claim 1, characterized in that electrolytes, in particular sodium chloride, are added to the aqueous phase, advantageously in concentrations of 0.1 - 5.0% by weight, particularly advantageously about 0.6 - 3.0% by weight, based on the total weight of the formulation.

14. Process for the preparation of W/O/W emulsions, characterized in that water containing 0.1 - 5% by weight (based on the total composition) of organic and/or inorganic electrolytes having mono- and/or divalent cations,
and an oily phase, this oily phase comprising

(a) at least one emulsifier (emulsifier A) chosen from the group consisting of emulsifiers of which the lipophilicity increases with increasing temperature and the hydrophilicity increases with decreasing temperature, the emulsifier or emulsifiers changing from an HLB value < 10 to an HLB value > 10 in the temperature range of 40 - 90°C, the HLB value of the emulsifier or emulsifiers at room temperature being between 11 and 18, preferably between 13 and 14, and the emulsifier not being completely soluble in the oily phase at about 80°C,
in particular at least one emulsifier of the general formula

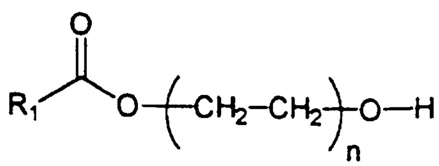 (emulsifier A),

in which

$R^1 = C_{10-30}$-alkyl and
n = a number from 8 to 200,

(b) if desired furthermore comprising:

(b1) one or more stabilizers of the general formula

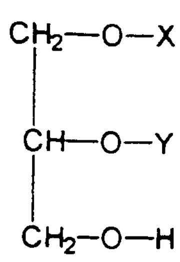

(stabilizer 1),

in which one of the radicals X or Y is a hydrogen atom and the remaining radical Y or X is chosen from the group consisting of branched or unbranched acyl radicals having 10 - 30 carbon atoms, and or

(b2) one or more branched and/or unbranched aliphatic fatty alcohols having 8 to 18 carbon atoms, in particular having 16 carbon atoms, that is to say cetyl alcohol,

(c) the base components of this oily phase, which are chosen from the group consisting of physiologically tolerated oils, fats and waxes, where

- the single base constituent of the oily phase, if this comprises only a single oil component, or
- the combination of the base constituents of the oily phase, if the oily phase comprises several oil components

has a "required HLB value (RHLB) of 10 - 20, and/or a polarity (determined in units of surface tension) of less than 30 mN/m,

are brought together at a temperature at which the oily phase and the aqueous phase are essentially in liquid form, and the mixture thus formed is agitated constantly and if appropriate subjected to one or more homogenizing steps, and the W/O/W emulsion thus formed is then allowed to cool to room temperature.

**15.** Process according to Claim 14, characterized in that the W/O/W emulsion is prepared at a temperature above the PIT and is then brought to a temperature below the PIT.

## Revendications

**1.** Emulsions multiples du type E/H/E, constituées d'une phase aqueuse externe continue, d'une phase huileuse qui y est dispersée et d'une deuxième phase aqueuse (interne) dispersée dans cette phase huileuse contenant

(a) au moins un émulsifiant (émulsifiant A), choisi parmi le groupe des émulsifiants dont la lipophilie augmente avec une température croissante et l'hydrophilie augmente avec une température décroissante, l'émulsifiant ou les émulsifiants changeant d'un indice de HLB < 10 à un indice HLB > 10 dans la gamme de températures de 40-90°C, la valeur de HLB de l'émulsifiant ou des émulsifiants à la température ambiante étant comprise entre 11 et 18, de préférence entre 13 et 14, et l'émulsifiant n'étant pas totalement soluble dans la phase huileuse lorsque la phase huileuse est combinée avec la phase aqueuse,
(b) une phase huileuse, les constituants de base de la phase huileuse étant choisis parmi le groupe constitué des huiles, matières grasses et cires physiologiquement acceptables, où

- le constituant de base unique de la phase huileuse, si celle-ci ne contient qu'un composant huileux unique, ou
- la combinaison des constituants de base de la phase huileuse, si la phase huileuse contient plusieurs composants huileux

présente un "indice HLB requis" (HLBR) de 10-20, et/ou une polarité, (déterminée en unités de tension superficielle) inférieure à 30 mN/m.
(c) une phase aqueuse externe et une phase aqueuse interne, ces phases aqueuses contenant 0,1-5% en poids (par rapport à la composition totale) d'électrolytes organiques et/ou inorganiques à cations mono-, di- ou trivalents,
(d) si on le souhaite, des auxiliaires et/ou additifs supplémentaires, dont le but principal est de stabiliser les gouttes d'émulsions multiples après leur formation in situ, et qui peuvent être incorporés dans la phase huileuse et/ou les phases aqueuses,
(e) si on le souhaite, des substances de base, auxiliaires, additifs et/ou substances actives supplémentaires courants en cosmétique ou en galénique médicale, qui peuvent être incorporés dans la phase huileuse et/ou les phases aqueuses.

**2.** Emulsions selon la revendication 1, caractérisées en ce que l'émulsifiant ou les émulsifiants A sont choisis parmi le groupe constitué des émulsifiants de formule générale

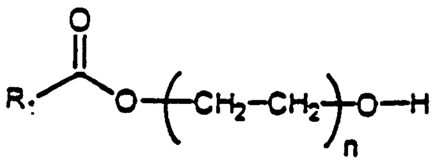

(émulsifiant A),

dans laquelle

$R_1$ = alkyle en $C_{10-30}$
n = un nombre allant de 8 à 200.

**3.** Emulsions selon la revendication 2, caractérisées en ce que l'émulsifiant ou les émulsifiants A sont choisis parmi le groupe dans lequel n prend des valeurs allant de 20 à 40.

**4.** Emulsions selon la revendication 1, caractérisées en ce que l'émulsifiant ou les émulsifiants A sont choisis parmi le groupe constitué par

(a) les mélanges de lécithine, d'alcools gras et d'acides gras tels qu'ils sont disponibles, par exemple, sous la dénomination commerciale Biophilic®S,
(b) le laurate de sucrose, tel qu'il est disponible par exemple, sous la dénomination commerciale Grilloten® K87,
(c) des esters de monoacides gras avec du polyglycérol, par exemple l'isostéarate de polyglycérol, tel qu'il est disponible sous la dénomination commerciale Polydermanol® GE-14, ainsi que le mono-isostéarate de décaglycéryle tel qu'il est disponible sous la dénomination Decaglyn® 1-IS,
(d) des diméthicone-copolyols tels qu'ils sont disponibles sous la dénomination DC 193.

**5.** Emulsions selon la revendication 1, contenant un ou plusieurs stabilisants de formule générale (1)

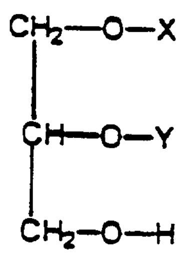

dans laquelle l'un des radicaux X ou Y représente un atome d'hydrogène et le radical Y ou X restant étant choisi parmi le groupe constitué des radicaux acyle ramifiés ou non ramifiés ayant 10-30 atomes de carbone.

**6.** Emulsions selon la revendication 5, caractérisées en ce que le monostéarate de glycérol est choisi en tant que stabilisant 1.

**7.** Emulsions selon la revendication 6, caractérisées en ce que l'émulsifiant ou les émulsifiants A et/ou le stabilisant ou les stabilisants 1 sont utilisés en concentrations de 0,05-15,0% en poids, de préférence en concentration de 0,1-7,5% en poids, particulièrement préférablement en concentration de 0,5-5,0% en poids, chaque fois par rapport au poids total des formulations.

**8.** Emulsions selon la revendication 1 caractérisées en ce que la phase huileuse est choisie parmi le groupe des huiles des familles de plantes des Euphorbiacées, Poacées, Fabacées, Bracicacées, Pédalacées, Astéracées, Linacées, Flacourticacées, et Violales, de préférence choisies parmi le groupe constitué de l'huile de ricin, de l'huile de germe de blé, de l'huile de pépins- de raisin, de l'huile de coprah, de l'huile de carthame, de l'huile de chardon, de l'huile d'onagre présentes dans la nature et d'autres huiles, qui contiennent au moins 1,5% en poids de glycérides d'acide linoléique.

**9.** Emulsions selon la revendication 1, caractérisées en ce que la phase huileuse est choisie parmi le groupe constitué de composants huileux qui présentent une polarité comprise entre 10 et 30 mN/m.

**10.** Emulsions selon la revendication 1, caractérisées en ce que les composants huileux sont choisis parmi le groupe constitué d'huiles qui présentent une polarité comprise entre 10 et 20 mN/m.

**11.** Emulsions selon la revendication 1, caractérisées en ce qu'un ou plusieurs alcools gras et/ou acides gras aliphatiques ramifiés et/ou non ramifiés, ayant de 8 à 18 atomes de carbone sont incorporés dans la phase huileuse.

**12.** Emulsions selon la revendication 1, caractérisées en ce que la phase huileuse ne contient pas, dans la phase huileuse, de composants présentant un point de fusion inférieur à 40°C.

**13.** Emulsions selon la revendication 1, caractérisées en ce que des électrolytes, en particulier du chlorure de sodium, sont ajoutés à la phase aqueuse, avantageusement en concentrations de 0,1-5,0% en poids, particulièrement avantageusement d'environ 0,6-3,0% en poids, par rapport au poids total de la formulation.

**14.** Procédé de préparation d'émulsions E/H/E, caractérisé en ce que de l'eau contenant 0,1-5% en poids (par rapport à la composition totale) d'électrolytes organiques et/ou inorganiques à cations mono- et/ou divalents,
et une phase huileuse, cette phase huileuse contenant

(a) au moins un émulsifiant (émulsifiant A), choisi parmi le groupe des émulsifiants dont la lipophilie augmente avec une température croissante et l'hydrophilie augmente avec une température décroissante, l'émulsifiant ou les émulsifiants changeant d'un indice de HLB < 10 à un indice HLB > 10 dans la gamme de températures de 40-90°C, la valeur de HLB de l'émulsifiant ou des émulsifiants à la température ambiante étant comprise entre 11 et 18, de préférence entre 13 et 14, et l'émulsifiant n'étant pas totalement soluble dans la phase huileuse à environ 80°C, en particulier au moins un émulsifiant de formule générale

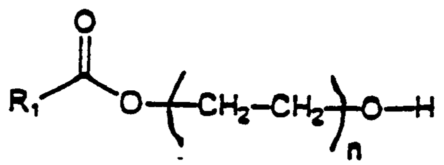
(émulsifiant A),

dans laquelle

$R_1$ = alkyle en $C_{10-30}$
n = un nombre allant de 8 à 200.

(b) contenant en outre éventuellement

(b1) un ou plusieurs stabilisants de formule générale

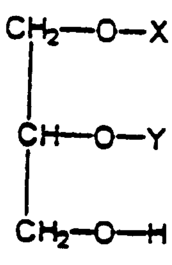
(stabilisant 1),

dans laquelle l'un des radicaux X ou Y représente un atome d'hydrogène et le radical Y ou X restant étant choisi parmi le groupe constitué des radicaux acyle ramifiés ou non ramifiés ayant 10-30 atomes de carbone, et/ou

(b2) un ou plusieurs alcools gras aliphatiques ramifiés et/ou non ramifiés, ayant de 8 à 18 atomes de carbone, ayant en particulier 16 atomes de carbone c'est-à-dire l'alcool cétylique

(c) les composants de base de cette phase huileuse qui sont choisis parmi le groupe constitué des huiles, matières grasses et cires physiologiquement acceptables, où

- le constituant de base unique de la phase huileuse, si celle-ci ne contient qu'un composant huileux unique, ou
- la combinaison des constituants de base de la phase huileuse, si la phase huileuse contient plusieurs composants huileux

présente un "indice HLB requis" (HLBR) de 10-20, et/ou une polarité, (déterminée en unités de tension superficielle) inférieure à 30 mN/m,

sont portés conjointement à une température à laquelle la phase huileuse et la phase aqueuse se trouvent essentiellement sous forme liquide et le mélange ainsi formé, est agité constamment et éventuellement soumis à une ou plusieurs étapes d'homogénéisation, et on laisse l'émulsion E/H/E ainsi formée refroidir jusqu'à température ambiante.

**15.** Procédé selon la revendication 14, caractérisé en ce que l'émulsion E/H/E est préparée à une température supérieure à la TIP et est ensuite amenée à une température inférieure à la TIP.

Fig. 1

W/O/W-Emulsion